# EUROPÄISCHE PATENTSCHRIFT

(11) **EP 2 439 721 B1**
(45) Veröffentlichungstag und Bekanntmachung des Hinweises auf die Patenterteilung: **19.07.2017**
(21) Anmeldenummer: 11183989.0
(22) Anmeldetag: 05.10.2011
(51) Int. Cl.: G09F 3/18, G09F 3/20, G09F 23/00, G09F 9/35, A61M 15/00

(54) **Austragvorrichtung für pharmazeutische Medien**
Discharge device for pharmaceutical media
Dispositif d'évacuation de milieux pharmaceutiques

(30) Priorität: 05.10.2010 DE 102010042007
(43) Veröffentlichungstag der Anmeldung: 11.04.2012
(73) Patentinhaber: Aptar Radolfzell GmbH, 78315 Radolfzell (DE)
(72) Erfinder: Körner, Joachim, 88690 Uhldingen-Mühlhofen (DE); Kohnle, Jörg, 78056 VS-Schwenningen (DE); Lindenau, Klaus, 78315 Radolfzell (DE)
(74) Vertreter: Patentanwaltskanzlei Cartagena

(56) Entgegenhaltungen:
- WO-A2-2009/136020
- CN-A- 101 438 327
- US-A- 5 363 842
- US-A1- 2006 011 651
- US-A1- 2007 056 871
- US-A1- 2007 084 462

## Beschreibung

### ANWENDUNGSGEBIET UND STAND DER TECHNIK

Die Erfindung betrifft eine Austragvorrichtung für pharmazeutische Medien, insbesondere für pharmazeutische Flüssigkeiten, mit einem Gehäuse, welches einen Aufnahmeraum zur Aufnahme eines Mediumspeichers zumindest zum Teil umgibt, mit einer am Gehäuse vorgesehenen Austragöffnung zur Verabreichung des Mediums aus dem Mediumspeicher und mit einer elektronischen Schaltung, die zur Erfassung von Austragvorgängen ausgebildet ist. Dabei weist das Gehäuse bei gattungsgemäßen Austragvorrichtungen eine zumindest abschnittsweise konvex gewölbte Außenform auf.

Gattungsgemäße Austragvorrichtungen sind aus dem Stand der Technik allgemein bekannt. Sie können von Benutzern verwendet werden, um sich selbst Medikamente, insbesondere in flüssiger Form, zu verabreichen. Üblicherweise handelt es sich bei gattungsgemäßen Austragvorrichtungen um kleine Austragvorrichtungen mit einem Gesamtgewicht von weniger als 200 g. Diese Austragvorrichtungen werden je nach Typ häufig vom Benutzer mitgeführt. Dies gilt insbesondere für Austragvorrichtungen für solche Medikamente, die regelmäßig eingenommen werden müssen, wie beispielsweise bei Asthma-Sprays. Dass gattungsgemäße Austragvorrichtungen häufig vom Benutzer mitgeführt werden, führt zur Anforderung, dass solche Austragvorrichtungen so klein als möglich ausgebildet sein sollten.

Gattungsgemäße Austragvorrichtungen weisen eine elektronische Schaltung auf, die dafür ausgebildet ist, Austragvorgänge zu erfassen. So ist es mittels einer solchen elektronischen Schaltung möglich, dem Benutzer Informationen darüber zur Verfügung zu stellen, wie viele Austragvorgänge bereits durchgeführt wurden oder wie viele Austragvorgänge mit dem derzeit eingesetzten Medienspeicher noch möglich sind. Die Zurverfügungstellung einer solchen elektronischen Schaltung, die zur Zählung der Austragvorgänge ausgebildet ist, ist nicht rein optional, sondern in manchen Ländern Ergebnis einer gesetzlichen Vorgabe. Zählvorrichtungen für Austragvorrichtungen sind beispielsweise aus der WO 1991/06334 A1, der WO 1996/00595 A1 und der WO 2005/009325 A2 bekannt. Die elektronischen Schaltungen, die für die Zählvorgänge erforderlich sind, sind bei diesen und anderen aus dem Stand der Technik bekannten Austragvorrichtungen mit einer starren Leiterplatte realisiert. Eine üblicherweise ebenfalls bei solchen Austragvorrichtungen zu findende Anzeigevorrichtung ist bei Vorrichtungen des Standes der Technik üblicherweise als LC-Display mit einer planen Oberfläche realisiert. Der Anzeigebereich dieser LC-Displays ist meist sehr klein.

Aus dem Stand der Technik sind weiter beispielsweise aus US 2007/0056871 A1 Verpackungen für medizinische Produkte bekannt, wobei ein Aktivdisplay vorgesehen ist, an welchem Informationen anzeigbar sind. US 5 363 842 offenbart die Merkmale des Einleitungsteils des Anspruchs 1. Die Gestaltungsfreiheit bei der Entwicklung von Austragvorrichtungen wird durch diese genannten Komponenten, insbesondere also die starre und plane Leiterplatte als auch das starre und plane Display merklich eingeschränkt. Die hiermit versehenen Austragvorrichtungen weisen zumeist eine deutlich größere Außenform als Austragvorrichtungen ohne solche elektronischen Komponenten auf. Dies ist insbesondere bei Austragvorrichtungen störend, die bestimmtungsgemäß vom Benutzer stets mitgeführt werden, wie es beispielsweise bei so genannten MDIs (Metered-Dose Inhaler) der Fall ist.

### AUFGABE UND LÖSUNG

Aufgabe der Erfindung ist es daher, gattungsgemäße Austragvorrichtungen dahingehend weiterzubilden, dass diese einen kompakteren Aufbau aufweisen und dennoch die gewünschte und zum Teil gesetzlich geforderte Zählfunktionalität zur Verfügung stellen.

Diese Aufgabe wird gelöst durch den Gegenstand mit den Merkmalen des Anspruchs 1.

Gemäß Anspruch 1 ist vorgesehen, dass die elektronische Schaltung mit einer Anzeigevorrichtung verbunden ist, die einen Anzeigenbereich zur Darstellung von Statusinformationen aufweist, wobei dieser Anzeigebereich entsprechend der konvex gewölbten Außenform des Gehäuses eine gebogene Formgebung aufweist.

Zudem ist vorgesehen, dass die elektronische Schaltung eine gewölbte Leiterplatte aufweist, die hinter einer gewölbten Außenwandung des Gehäuses angeordnet ist und entsprechend der gewölbten Außenwandung eine gebogene Formgebung aufweist.

Die Außenform des Gehäuses, die beispielsweise im Wesentlichen rotationssymmetrisch sein kann und dadurch zumindest abschnittsweise gewölbt ist, setzt sich somit in gleicher Formgebung im Anzeigebereich der Anzeigevorrichtung fort. Somit kann gleichzeitig eine große Anzeigefläche erzielt werden, ohne dass diese für sich genommen bereits eine Vergrößerung des Gehäuses in seiner Gesamtheit erzwingen würde.

Ähnliches gilt für die elektronische Schaltung, die definitionsgemäß mindestens einen Mikroprozessor oder einen einfachen Controller zur Erfassung und Zählung von Austragvorgängen aufweist. Dabei ist die Verwendung einer gewölbten Leiterplatte von Vorteil, welche hinter einer gewölbten Außenwandung des Gehäuses angeordnet ist und der Wölbung der Außenwandung entsprechend gebogen ist, so dass auch auf diese Weise auf der Innenseite der Außenwandung des Gehäuses nur ein sehr geringer Bauraum gebraucht wird. Die Außenwandung, hinter der die mindestens eine Leiterplatte der elektronischen Schaltung angeordnet ist, kann auch durch die außenseitig von ihr angeordnete Anzeigevorrichtung gebildet sein.

Der gebogene Anzeigebereich der Anzeigevorrichtung und/oder die gebogene Leiterplatte können bereits im Zustand vor ihrer Montage die jeweilige gebogene Formgebung aufweisen, beispielsweise in dem sie jeweils ein bereits während der Herstellung in eine gebogene Stellung gebrachtes, beispielsweise tiefgezogenes, Kunststoffsubstrat umfassen. Alternativ zu einer solchen starr gebogenen Formgebung kann es sich jedoch auch um lediglich flexibel biegbare und ggf. beim Wegfall einer Biegekraft plane Komponenten handeln, welche im Zuge der Montage derartig am gewölbten Gehäuse der Austragvorrichtung festgelegt werden, dass sie dessen Wölbung ebenfalls annehmen. Hierzu ist es im Falle der Anzeigevorrichtung beispielsweise möglich, diese an einer Außenfläche des Gehäuses anzukleben und sie dadurch in die gebogene Formgebung zu zwingen. In Hinblick auf die Leiterplatte kann es zweckmäßig sein, diese an der Innenseite des Außengehäuses festzukleben und ihr dadurch in etwa die Formgebung des Gehäuses in diesem Bereich zu geben. Der Vorteil einer flexibel biegbaren Anzeigevorrichtung liegt auch darin, dass diese bei verschiedenen Modellen von Austragvorrichtungen mit einer voneinander abweichenden Wölbung der Außenwandung Verwendung finden kann.

Der mittlere Biegeradius der gebogenen Anzeigevorrichtung und/oder der gebogenen Leiterplatte beträgt vorzugsweise nicht mehr als 15 cm, insbesondere vorzugsweise nicht mehr als 8 cm oder sogar maximal 3 cm.

Die Anzeigevorrichtung weist ein Substrat auf, auf welchem Anzeigesegmente vorgesehen sind, die durch Drucktechnik oder Beschichtungstechnik auf diesem aufgebracht wurden.

Die Aufbringung von Anzeigesegmenten auf einem zur Anzeigevorrichtung gehörenden Substrat stellt eine sehr günstige Möglichkeit zur Herstellung einer Anzeigevorrichtung dar, welche in Hinblick auf einen erforderlichen günstigen Herstellungspreis von Vorteil ist. Die Anzeigesegmente können auf der Anzeigevorrichtung die Form einer Pixelmatrix ergeben, um beliebige und gegebenenfalls auch graphisch aufbereitete Informationen wiedergeben zu können. Bevorzugt ist es jedoch, wenn eine geringe Zahl von Anzeigesegmenten, vorzugsweise weniger als 100 Anzeigesegmenten Verwendung findet, da so eine kostengünstigere Herstellung sowohl der Anzeigevorrichtung als auch der zur Ansteuerung erforderlichen elektronischen Schaltung möglich ist. Insbesondere ist von Vorteil, wenn eine oder mehrere Sieben-Segmentanzeigen Verwendung finden. Zusätzlich kann zur schnelleren Erfassbarkeit der bereits ausgegebenen Medienmenge oder der noch enthaltenen Medienmenge auch ein aus einer Vielzahl von Anzeigesegmenten zusammengesetzter Balken vorgesehen sein. Die Anzeigesegmente werden durch elektrophoretische oder elektrochromatische Materialien gebildet. Sie können beispielsweise aus organischen Polymerverbindungen bestehen, die bereits aus dem OLED-Bereich bekannt sind und die beim Anlegen einer elektrischen Spannung leuchten. Alternativ hierzu ist auch eine Anzeigevorrichtung nach der E-Ink-Technologie denkbar. Auch LC-Displays können in insbesondere starr gebogener Ausgestaltungen Verwendung finden.

Die Aufbringung des Materials, welches die Anzeigesegmente bildet, erfolgt im Wege der Drucktechnik oder Beschichtungstechnik. Die Verbindung zwischen den Anzeigesegmenten und der elektronischen Schaltung zur Ansteuerung der Segmente erfolgt vorzugsweise über metallische Leiter oder Kunststoffleiter, die auch im Wege der Drucktechnik oder Beschichtungstechnik aufgebracht sein können.

Von Vorteil ist die Verwendung eines dünnen Kunststoffsubstrates, welches vorzugsweise eine Dicke zwischen 50 µm und 500 µm aufweist. Dieses Kunststoffsubstrat kann starr plan sein oder auch starr gebogen ausgebildet sein. Insbesondere kann es sich um eine Folie mit der genannten Materialstärke handeln. Als Material kommen jegliche nicht leitende Kunststoffe in Frage. Insbesondere bevorzugt wird die Verwendung von Polyester, von Polyamid oder von Polyimid.

Vorzugsweise weist die Anzeigevorrichtung neben dem Substrat eine weitere Kunststoffschicht auf, die die Anzeigesegmente auf der gegenüberliegenden Seite überdeckt. Das Grundsubstrat, auf dem die Anzeigesegmente aufgebracht sind und die weitere Kunststoffschicht sind zumindest im Randbereich, vorzugsweise jedoch flächig, miteinander verbunden, um das Eintreten von Flüssigkeit oder Luft in die Anzeigevorrichtung zu vermeiden.

Bevorzugt ist eine formflexible Leiterplatte insbesondere eine als FPCB-Leiterplatte ausgebildete Leiterplatte. Die Leiterbahnen können auf dem Kunststoffsubstrat aufgedruckt sein oder im erhitzten Zustand aufgepresst werden, um eine innige Verbindung mit dem Kunststoffsubstrat einzugehen. Im Falle von aufgedruckten Leiterbahnen können diese insbesondere aus leitfähigen organischen Polymeren oder aus Metall sein.

Die elektronische Schaltung weist mindestens einen Mikroprozessor sowie ggf. zusätzlich einen Multiplexer zur Ansteuerung der Anzeigevorrichtung auf. Dieser Mikroprozessor sowie weitere Bauteile, wie beispielsweise ein Speicher oder der Multiplexer, können als diskrete Bauteile ausgebildet sein, die durch das Kunststoffsubstrat hindurch oder durch eine gegenüberliegend aufgebrachte Folie hindurch mit den Leiterbahnen kontaktiert sind. Eine bevorzugte alternative Ausgestaltung sieht vor, dass der für ein Zählwerk sehr einfach ausgebildete Mikroprozessor zur Erfassung von Austragvorgängen und/oder zur Ansteuerung der Anzeigevorrichtung mittels Drucktechnik auf dem Kunststoffsubstrat aufgebracht ist. Dies ermöglicht eine sehr flache Bauweise.

Die erfindungsgemäße Austragvorrichtung weist vorzugsweise weiterhin eine Batterie zur Versorgung der elektrischen Schaltung und/oder der Anzeigevorrichtung mit elektrischer Energie auf, wobei diese Batterie vorzugsweise als Flachbatterie ausgebildet ist und hinter einer gewölbten Außenwandung des Gehäuses angeordnet ist, wobei sie entsprechend dieser gewölbten Außenwandung eine gebogene Formgebung aufweist. Auf diese Weise nimmt auch die Batterie selbst nur einen minimalen Bauraum in der Austragvorrichtung ein und kann an einer Außenwandung anliegen oder nahezu anliegend positioniert werden. Vorzugsweise handelt es sich um eine Folienbatterie, welche beidseitig durch Kunststofffolien verschlossen ist. Diese Batterie verfügt vorzugsweise über mindestens gedruckt aufgebrachte zwei Lagen zwischen den Kunststofffolien, wobei in einer Lage die Elektroden der Batterie angeordnet sind und wobei in einer darüber oder darunter angeordneten Lage eine Elektrolytschicht aufgebracht ist. Auch ist es möglich, die beiden Elektroden beidseitig der Elektrolytschicht vorzusehen, so dass inklusive der beiden die Batterie nach oben und unten abschließenden Folien ein insgesamt mindestens fünflagiger Schichtverbund entsteht.

Weiterhin weist die Austragvorrichtung vorzugsweise einen Sensor zur Erfassung eines Austragvorgangs auf. Dieser Sensor ist insbesondere vorzugsweise als Kraftsensor oder kapazitiver Sensor ausgebildet. Vorzugsweise ist auch der Sensor auf einem Kunststoffsubstrat aufgedruckt.

Insbesondere von Vorteil ist es, wenn der Sensor derart ausgebildet ist, dass er die Bewegung des Medienspeichers im Aufnahmeraum erfassen kann. Bei vielen Austragvorrichtungen wird der Medienspeicher verlagert, um dadurch einen Austragvorgang zu bewirken. Diese Verlagerung kann beispielsweise dadurch erfasst werden, dass der Medienspeicher eine Kraft auf einen Abschnitt des Sensors ausübt. Diese Kraft kann erfasst werden. So ist es beispielsweise möglich, einen Dehnungsmessstreifen oder einen Piezosensor auf einem Kunststoffsubstrat aufzubringen, insbesondere aufzudrucken, der bei einer Verformung des Kunststoffsubstrates eine über einen Mikroprozessor messbare Spannungsveränderung oder Widerstandsveränderung bewirkt. Dieser Dehnungsmessstreifen bzw. dieser Piezosensor ist vorzugsweise ebenfalls zwischen zwei Lagen Kunststofffolie angeordnet. Ein hierzu alternativer kapazitiver Sensor kann ebenfalls per Drucktechnik aufgebracht werden wie es bei kapazitiven Bildschirmen heutiger Mobiltelefone bereits üblich ist. Durch ihn ist es möglich, eine Bewegung des gegenüber dem Sensor bewegten Medienspeichers im Aufnahmeraum zu erfassen.

Das Gehäuse der Austragvorrichtung weist bei einer Ausgestaltung eine nach außen gewandte Außenschale und eine zum Aufnahmeraum gewandte Innenschale auf, die gemeinsam einen dazwischen liegenden Elektronikraum begrenzen, wobei in diesem Elektronikraum die Anzeigevorrichtung und/oder die mindestens eine Leiterplatte angeordnet ist. Dieser Elektronikraum, der eine sehr geringe lichte Breite von beispielsweise unter 3mm haben kann, hat die gewölbte Formgebung des Gehäuses in diesem Bereich. Die Verwendung der oben beschriebenen Komponenten (Anzeigevorrichtung, elektronische Schaltung, Sensor, Batterie) lässt auch diesen geringen Bauraum zur Anordnung ausreichend sein. Vorzugsweise handelt es sich um einen ringförmigen Elektronikraum, der den Aufnahmeraum getrennt durch die Innenschale umgibt.

Bei einer hierzu alternativen Gestaltung weist das Gehäuse lediglich eine nach außen gewandte Außenschale auf, an deren Innenseite die mindestens eine Leiterplatte oder die Flachbatterie vorgesehen ist, wobei zwischen der Leiterplatte und dem Aufnahmeraum zur Aufnahme des Medienspeichers keine weiteren Wandungen des Gehäuses vorgesehen sind. Bei einer solchen Gestaltung liegt somit eine Außenwandung des Medienspeichers der Innenseite der Leiterplatte unmittelbar gegenüber. Eine besonders schlanke Bauweise der Austragvorrichtung im Bereich der Leiterplatte bzw. der Batterie ist hierdurch erzielbar.

Alternativ zu der Anordnung zumindest der Leiterplatte innerhalb des Gehäuses ist es auch möglich, dass eine flexible Baueinheit umfassend die Anzeigevorrichtung, die elektronische Schaltung, den Sensor und die Batterie außenseitig auf eine Außenwandung des Gehäuses aufgebracht ist. Dies gestattet es, eine erfindungsgemäße Austragvorrichtung gegenüber einer bislang üblichen Austragvorrichtung ohne elektronische Komponenten hinsichtlich des Gehäuses weitgehend baugleich zu gestalten. Auf der erfindungsgemäßen Austragvorrichtung wird lediglich außenseitig der vorzugsweise in sich flexible Verbund aus den genannten Komponenten aufgebracht. Dieser kann zu diesem Zweck insbesondere festgeklebt werden, wobei es besonders vorteilhaft ist, wenn der Verbund auf einer Innenseite eine Selbstklebefläche aufweist, mittels derer die Anbringung an dem Gehäuse der Austragvorrichtung möglich ist. Da nach derzeitigem Stand der Technik der jeweiligen elektronischen Komponenten eine Dicke von etwa zwei bis drei mm für diesen Verbund schwer zu unterschreiten ist, ist vorzugsweise an der Außenwandung des Gehäuses eine Vertiefung vorgesehen, in die der Verbund eingesetzt wird, so dass er nicht durch eine exponierte Anordnung besonders beschädigungsgefährdet ist.

Die Anzeigevorrichtung und/oder die Außenwandung, hinter der die mindestens eine flexible Leiterplatte angeordnet ist, sind vorzugsweise im Bereich eines zylindrischen Gehäuseabschnitts vorgesehen, wobei die Querschnittsform dieses zylindrischen Gehäuseabschnitts vorzugsweise eine Kreisform bildet. Ein solcher zylindrische Gehäuseabschnitt, insbesondere ein kreiszylindrischer, findet sich bei vielen Austragvorrichtungen die zumindest abschnittsweise rotationssymmetrisch aufgebaut sind. Die Anbringung der Anzeigevorrichtung bzw. der Leiterplatte in einem solchen zylindrischen Abschnitt führt dazu, dass ein Verbiegen jeweils nur in einer Biegeebene erfolgen muss, was die Herstellbarkeit einer solchen Leiterplatte bzw. einer solchen Anzeigevorrichtung erleichtert. Alternativ zu einer kreiszylindrischen Form kann es jedoch auch von Vorteil sein, wenn eine hiervon abweichende Formgebung in dem Gehäuseabschnitt vorliegt, an dem die Anzeigevorrichtung oder die Leiterplatte angeordnet werden. Insbesondere ist es von Vorteil, wenn die Anzeigevorrichtung und/oder die Leiterplatte im Bereich einer Außenwandung vorgesehen sind, deren Krümmungsradius größer als der maximale Halbdurchmesser des Gehäuses in diesem Gehäuseabschnitt ist. Dies kann beispielsweise durch einen elliptischen Querschnitt des Gehäuses in diesem Gehäuseabschnitt gegeben sein. Auch ist eine partielle Abflachung des Querschnitts an jener Seite, an der die Anzeigevorrichtung und/oder die Leiterplatte vorgesehen sein sollen, möglich. Diese von der kreiszylindrischen Form abweichende Ausgestaltung des Gehäuseabschnitts erlaubt die Vermeidung geringer Biegeradien hinsichtlich der Anzeigevorrichtung und/oder der Leiterplatte.

Wie oben dargelegt ist, sind alle vier wesentlichen Komponenten der Elektronik einer erfindungsgemäßen Austragvorrichtung, also der Anzeigevorrichtung, der elektronischen Schaltung mit Mikroprozessor, des Sensors zur Erfassung eines Austragvorgangs und der Batterie auf Basis eines flexibel biegbaren Substrates möglich, auf dem die entsprechenden Komponenten zumindest zum Teil drucktechnisch aufgebracht werden können. Ein erheblicher Kostenvorteil bei der Herstellung ergibt sich daraus, wenn zumindest zwei dieser Komponenten ein gemeinsames und einstückiges Kunststoffsubstrat teilen. So kann insbesondere der Sensor aus dem gleichen Kunststoffsubstrat aufgebracht sein wie die elektronische Schaltung bzw. die Leiterbahnen der elektronischen Schaltung von diskret darauf angeordneten Bauelementen wie einem Mikroprozessor. Auch können diese beiden Komponenten bzw. eine von ihnen aus dem gleichen Kunststoffsubstrat aufgebracht sein, welches gleichzeitig der Aufbringung der elektrochromatischen oder elektrophoretischen Materialien zur Bildung von Anzeigesegmenten dient. Besonders von Vorteil ist es, wenn zumindest zwei der ein gemeinsames Substrat aufweisenden Komponenten auch eine gemeinsame dem Substrat gegenüberliegende Deckfolie aufweisen.

Es ist von besonderem Vorteil, wenn von den genannten vier Komponenten mindestens drei entlang eines flexibel verformbaren Bauteilstrangs hintereinander angeordnet sind, wobei dieser Bauteilstrang der Formgebung des zylindrischen Gehäuseabschnitts folgend und vorzugsweise über einen Umfang des zylindrischen Gehäuseabschnitts umfänglich verteilt im bzw. am Gehäuse angeordnet sind. Unter einem Bauteilstrang wird in diesem Zusammenhang ein Verbund aus mindestens drei der genannten Komponenten verstanden, zwischen denen jeweils ein formflexibler Abschnitt vorgesehen ist, wobei die mindestens zwei formflexiblen Verbindungsabschnitte zwischen den Komponenten in einer gemeinsamen Ebene biegbar sind. So kann der Strang auf einfache Weise und sehr platzsparend in bzw. an einer Austragvorrichtung angeordnet werden. Es ist dabei von Vorteil, wenn dieser Bauteilstrang, der im Bereich der Gehäusewandung vorgesehen ist, den umschlossenen Aufnahmeraum um mindestens 180°, vorzugsweise um 270°, umgibt. Hierdurch wird eine ideale Raumnutzung erzielt, da der Bauteilstrang aufgrund seiner großen umfänglichen Länge sehr dünn ausgebildet sein kann.

Alternativ zu einem entlang des Umfangs angeordneten Bauteilstrang ist es auch möglich, dass mindestens zwei der genannten Komponenten jeweils eine gebogene Formgebung aufweisen und nebeneinander liegend die Schichten eines gemeinsamen Schichtverbundes darstellen. In Radialrichtung des zylindrischen Gehäuseabschnitts weist ein solcher Schichtverbund somit mehrere Komponenten auf. So kann beispielsweise innenliegend eine flache Batterie oben beschriebener Bauart angeordnet sein. An diese schließt sich außenseitig die elektronische Schaltung mit der insbesondere flexiblen Leiterplatte an. Außenseitig ist an dieser wiederum die ebenfalls gebogene Anzeigevorrichtung vorgesehen. Dabei kann es sich um einen einfach oder mehrfach gefalteten Bauteilstrang mit diesen Komponenten handeln. Auch ist es möglich, dass die Komponenten schichtweise aufeinander aufgebaut sind und jeweils durch eine Kunststofffolie oder durch ein anderweitiges Substrat isolierend schichtweise aufeinander angeordnet sind.

Die erfindungsgemäße Gestaltung kann bei allen üblichen Sorten gattungsgemäßer Austragvorrichtungen Anwendung finden, so beispielsweise bei solchen mit einer integrierten Pumpe oder bei Squeezebottles. Insbesondere bevorzugt ist die Anwendung jedoch bei solchen Austragvorrichtungen, die als Inhalator mit einem in etwa zylindrischen Hauptabschnitt ausgebildet ist, an dessen Ende sich ein radial vom Hauptabschnitt weg erstreckendes Mundstück mit der Austragöffnung befindet. Dabei bildet der Hauptabschnitt den zylindrischen Gehäuseabschnitt zur Anordnung der Anzeigevorrichtung und/oder der mindestens einen Leiterplatte. Ein solcher Aufbau ist von sogenannten MDIs (Metered-Dose Inhaler) bekannt. Gerade bei dieser Art von Austragvorrichtungen sind die regulatorischen Anforderungen zur Anordnung einer Zählvorrichtung vergleichsweise hoch. Gleichzeitig sind es auch diese Austragvorrichtungen, die üblicherweise vom Benutzer mitgeführt werden und die daher möglichst klein ausgebildet sein sollten. Die Verwendung erfindungsgemäß angeordneter Leiterplatten, einer erfindungsgemäß gebogen ausgebildeten Leiterplatte und/oder einer erfindungsgemäß gebogenen Anzeigevorrichtung ist daher gerade im Hinblick auf diese Austragvorrichtungen von besonderem Vorteil.

### KURZBESCHREIBUNG DER ZEICHNUNGEN

Weitere Vorteile und Aspekte der Erfindung ergeben sich außer aus den Ansprüchen aus der nachfolgenden Beschreibung von bevorzugten Ausführungsbeispielen der Erfindung, die nachfolgend anhand der Figuren erläutert sind. Dabei zeigen:
- Figur 1: eine als MDI ausgebildete Austragvorrichtung mit und ohne eingesetzten Medienbehälter,
- Figur 2: die elektronischen Komponenten der Austragvorrichtung der Figur 1 in einem Vormontagezustand,
- Figur 3: die Austragvorrichtung der Figur 1 in einer teilgeschnittenen Ansicht,
- Figur 4: einen Querschnitt durch die Austragvorrichtung der Figur 1,
- Figuren 5a bis 5d: Querschnitte durch alternativ aufgebaute Austragvorrichtungen und
- Figur 6a und 6b: Alternative Ausgestaltungen einer erfindungsgemäßen Austragvorrichtung.

### DETAILLIERTE BESCHREIBUNG DER AUSFÜHRUNGSBEISPIELE

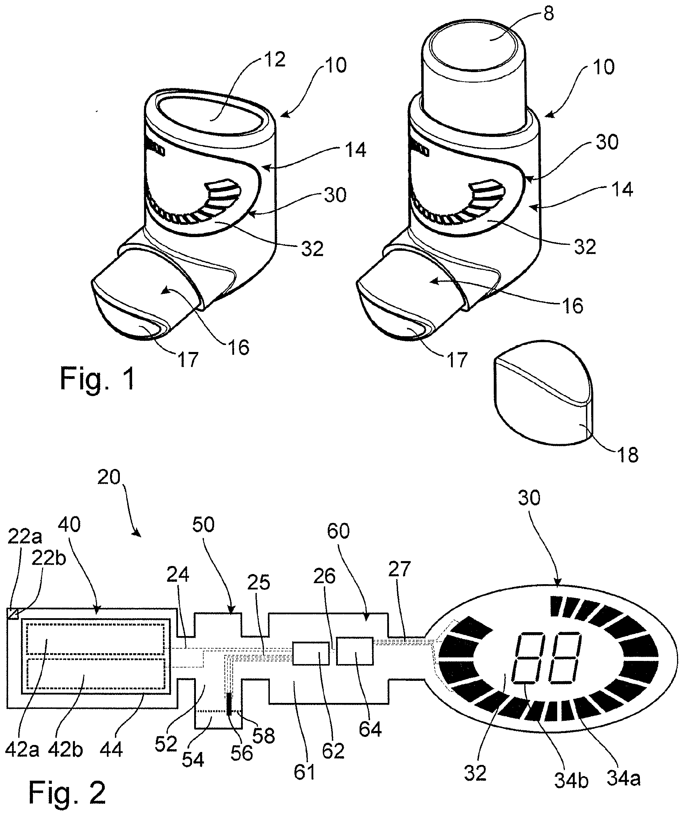

Fig. 1 zeigt eine erfindungsgemäße Austragvorrichtung 10, wobei in der rechten Darstellung bereits ein Medienspeicher 8 eingesetzt ist. Die linke Darstellung zeigt die Austragvorrichtung 10 ohne diesen Medienspeicher 8, so dass der für diese vorgesehene einseitig offene Aufnahmeraum 12 zu erkennen ist. Die Austragvorrichtung 10 verfügt über ein Gehäuse mit einem zylindrischen Gehäuseabschnitt 14, der diesen Aufnahmeraum 12 umgibt, sowie mit einem sich am unteren Ende daran anschließenden und vom Gehäuseabschnitt 14 radial nach außen erstreckenden Mundstück 16 mit Austragöffnung 17, welches bei Nichtgebrauch mittels einer Kappe 18 verschlossen werden kann.

Im Wesentlichen entspricht die Formgebung der Austragvorrichtung 10 in etwa üblichen MDIs (Metered Dose Inhaler). Abweichend von üblichen Gestaltungen weist die Austragvorrichtung 10 jedoch eine Anzeigevorrichtung 30 mit einem Anzeigenbereich 32 auf, der nicht plan ausgebildet ist, sondern der Außenfläche des zylindrischen Hauptabschnitts 14 des Gehäuses folgend gekrümmt ausgebildet ist und eine vergleichsweise große Fläche der dem Benutzer bei Benutzung zugewandten Seite des Hauptabschnitts 14 bedeckt.

Die Fig. 2 bis 4 verdeutlichen den Aufbau der Austragvorrichtung 10. Fig. 2 zeigt eine Elektronikbaugruppe 20, die in der Austragvorrichtung 10 Verwendung findet. Diese Elektronikbaugruppe ist als langgezogener Strang ausgebildet, der über seine gesamte Länge oberseitig und unterseitig flexible Kunststofffolien 22a, 22b aus Polyamid begrenzt ist. Die Zweilagigkeit der Folien ist zur Anschauung in der linken oberen Ecke verdeutlicht. Die strangförmige Baugruppe 20 weist bezogen auf die Fig. 2 von links nach rechts insgesamt vier Unterbaugruppen auf. Linksseitig ist eine Flachbatterie 40 vorgesehen. Im Bereich dieser Flachbatterie 40 sind auf der bezogen auf Fig. 2 unteren Folie 22a zwei Elektroden 42a, 42b vorgesehen. Über diesen Elektroden 42a, 42b erstreckt sich ein Elektrolyt 44 der Batterie 40, der ebenso wie die Elektroden 42a, 42b je nach Typ der Batterie jeweils verschieden ausgebildet sein kann. Die beiden Schichten, die durch die Elektroden 42a, 42b einerseits und den Elektrolyten 44 andererseits gebildet werden, sind umfänglich durch die Polyamidfolien 22a, 22b nach außen annähernd umfänglich abgeschlossen. Sowohl die Elektroden 42a, 42b als auch der Elektrolyt 44 sind mittels Drucktechnik auf der unteren Polyamidfolie 22a aufgebracht worden, bevor die obere Deckfolie 22b oberseitig angebracht wurde. Die Elektroden sind über ebenfalls drucktechnisch aufgebrachte Leiterbahnen 24 mit einem Mikroprozessor 62 verbunden. Dies wird im Weiteren noch erläutert. Rechtsseitig schließt sich an die Batterie 40 ein Sensor 50 an. Dieser Sensor 50 wird durch einen im Vormontagezustand der Fig. 2 zur Batterie ebenen parallelen Stützabschnitt 52 und einem sich daran anschließenden Sensorabschnitt 54 gebildet, wobei der Stützabschnitt 52 und der Sensorabschnitt 54 wiederum als zweilagiger Folienverbund aus den Polyamidfolien 22a, 22b ausgebildet sind. In nachfolgend noch erläuterter Weise ist der Sensorabschnitt 54 in einem Ruhezustand gegenüber dem Stützabschnitt 52 um etwa 30° angewinkelt. In einem Übergangsbereich 58 zwischen dem Sensorabschnitt 52 und dem Stützabschnitt 54 ist ein Dehnungsmessstreifen 56 vorgesehen, welcher über Leiterbahnen 25, die ebenfalls auf der unteren Polyamidfolie 22a drucktechnisch aufgebracht sind, ebenfalls mit dem Mikroprozessor 62 verbunden ist. Dieser Dehnungsmessstreifen 56 ist derart angeordnet, dass er in Abhängigkeit des Winkels zwischen dem Stützabschnitt 52 und dem Sensorabschnitt 54 einen variablen Widerstand darstellt, so dass eine Veränderung dieses Winkels für den Mikroprozessor 62 erfassbar ist. Alternativ zu dem Dehnungsmessstreifen 56 kann beispielsweise auch ein Piezosensor zwischen den Polyamidfolien 22a, 22b vorgesehen sein, der eine Biegung im Übergangsbereich 58 durch Abgabe einer verformungsabhängigen Spannung erfassbar macht, wobei der Vorteil gegenüber einem Dehnungsmessstreifen unter anderem in dem Wegfall der Notwendigkeit einer Brückenschaltung liegt. Rechtsseitig schließt sich an den Sensor 50 eine Steuerungselektronik 60 an. Diese Steuerungselektronik umfasst den bereits genannten Mikroprozessor 62 sowie einen mit diesem über Leiterbahnen 25 verbundenen Multiplexer 64. Der Mikroprozessor 62 sowie der Multiplexer 64, die als konventionell ausgebildete ICs auf Siliziumbasis ausgebildet sind, sind auf eine flexible Leiterplatte 61 aufgesetzt, die durch die Folien 22a, 22b und die dazwischenliegenden drucktechnisch aufgebrachten Leiterbahnen 24, 25, 26, 27 gebildet ist.

Der Multiplexer 64 ist über die Leiterbahnen 27, von denen nur einige beispielhaft dargestellt sind, mit der Anzeigevorrichtung 30 verbunden. Diese Anzeigevorrichtung 30 ist wie die vorgenannten Unterbaugruppen 40, 50, 60 ober- und unterseitig durch die Folien 22a, 22b begrenzt. Dazwischen sind Anzeigesegmente 34a, 34b vorgesehen, welche vorliegend in Form einer teilkreisförmigen Füllstandsbalkens 34a und zweier Sieben-Segmentanzeigen 34b angeordnet sind. Die insgesamt 34 Anzeigensegmente sind mittels einzelner Leiterbahnen 27 mit dem Multiplexer 64 verbunden, so dass sie durch den Mikroprozessor 62 in Verbindung mit dem Multiplexer 64 einzeln aktiviert und deaktiviert werden können.

Die dargestellte Baugruppe 20 ist herstellungstechnisch von großem Vorteil. Bis auf den Mikroprozessor 62 und dem Multiplexer 64 sind lediglich zwei Folien 22a, 22b vorgesehen, zwischen denen verschiedene drucktechnisch aufbringbare Funktionsbauteile angeordnet sind, die gemeinsam eine vollständige Elektronik für die Austragvorrichtung 10 bieten.

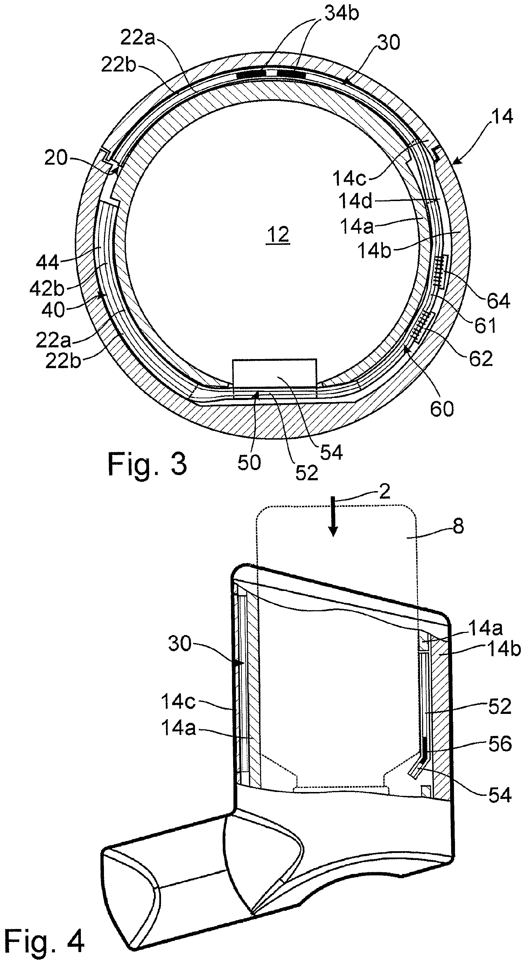

Die Fig. 3 und 4 erläutern, wie die Baugruppe 20 in der Austragvorrichtung 10 angeordnet ist. Bezugnehmend auf die Fig. 3, die eine Schnittdarstellung durch den zylindrischen Hauptabschnitt 14 der Austragvorrichtung zeigt, ist erkennbar, dass das starre Kunststoffgehäuse der Austragvorrichtung 10 in diesem Bereich im Wesentlichen aus einer Innenwandung 14a und einer Außenwandung 14b besteht. Die Außenwandung 14b weist im Bereich der Anzeigevorrichtung 30 eine Aussparung auf, in die ein transparentes Sichtfenster 14c eingesetzt ist. Zwischen der Innenwandung 14a die unmittelbar den Aufnahmeraum 12 zur Aufnahme des Medienspeichers 8 umgibt, und der Außenwandung 14b bzw. dem Sichtfenster 14c ist ein ringförmiger Aufnahmeraum 14d vorgesehen, in dem die Baugruppe 20 derart angeordnet ist, dass sie annähernd vollumfänglich den Aufnahmeraum 12 umgibt. Dabei ist die Anzeigevorrichtung 30 unmittelbar hinter dem Sichtfenster 10 vorgesehen. Im Uhrzeigersinn schließt sich an die Anzeigevorrichtung 30 die Steuerungselektronik-Unterbaugruppe 60 an, wobei die integrierten Schaltkreise, die der Mikroprozessor 62 und den Multiplexer 64 bilden, aufgrund ihrer flachen Bauweise unschwer in den Aufnahmeraum 14d hineinpassen. Weiter im Uhrzeigersinn schließt sich an die Steuerungselektronik-Unterbaugruppe die Sensor-Unterbaugruppe 50 an, wobei in der in Fig. 3 und insbesondere auch in Fig. 4 dargestellten Art und Weise zu erkennen ist, dass der Sensorabschnitt 54 durch ein Fenster in der Innenwandung 14a in den Aufnahmeraum 11 für den Medienspeicher 12 hineinragt. An die Sensor-Unterbaugruppe 50 wiederum schließt sich die Flachbatterie 40 an.

Wie aus der Fig. 3 gut ersichtlich ist, sind alle Unterbaugruppen 30, 40, 50, 60 des Bauteilstrangs 20 durch ihre Biegbarkeit und die Biegbarkeit der zwischen ihnen gelegenen verjüngten Verbindungsabschnitte gut im Aufnahmeraum 14d unterzubringen. Die Außenmaße der Austragvorrichtung 10 weichen daher kaum von den Außenmaßen eines MDIs ab, welcher ohne eine Zählelektronik ausgebildet ist.

Im Betrieb wird, wie anhand Fig. 4 erläutert ist, ein Austragvorgang dadurch bewirkt, dass der Medienspeicher 8 in Richtung des Pfeils 2 tiefer in den Aufnahmeraum 12 hineingedrückt wird. Hierbei kommt es zu einer Auslenkung des Sensorabschnitts 54 gegenüber dem Stützabschnitt 52 wodurch der Dehnungsmessstreifen 56, der im Übergangsbereich angeordnet ist, sich hinsichtlich seines Widerstandswertes verändert. Im Falle der Verwendung eines Piezosensors würde die Auslenkung zur Erzeugung einer messbaren Spannung führen. Dies ist durch den Mikroprozessor 62 erfassbar, so dass ein darin vorgehaltener Zähler für Austragvorgänge in Folge dieser erfassten Auslenkung um eins erhöht wird. Gleichzeitig wird auch vom Mikroprozessor 62 zusammen mit dem Multiplexer 64 eine Aktualisierung der Darstellung der Anzeigevorrichtung 30 vorgenommen.

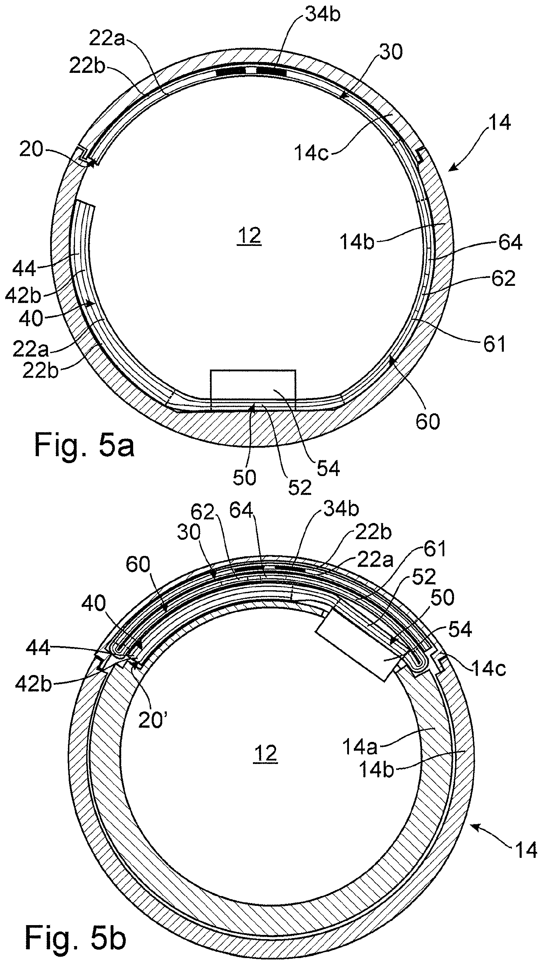

Die Fig. 5a bis 5d zeigen in einer Querschnittsdarstellung, die mit der Fig. 3 vergleichbar ist, alternative Ausgestaltungen der Austragvorrichtung 10. Nachfolgend werden lediglich die maßgeblichen Unterschiede erläutert. In den Fig. 5a bis 5d sind der Mikroprozessor 62 und der Multiplexer 64 als unmittelbar zwischen den Folienlagen aufgedruckte Bauteile ausgeführt. Diese Gestaltung ist optional. Bei den genannten Beispielen könnten gleichermaßen auch konventionelle diskrete Bauteile 62, 64 wie im Beispiel der Fig. 1 bis 4 Anwendung finden.

Die Ausgestaltung der Fig. 5a entspricht weitgehend der Ausgestaltung der Fig. 2 bis 4. Ein wesentlicher Unterschied besteht allerdings darin, dass bei dieser Ausgestaltung die Innenwandung 14a entfallen ist. Um diese zu kompensieren sind die Unterbaugruppen 30, 40, 50, 60 in nicht näher dargestellter Art und Weise zumindest zum Teil mittels einer Klebverbindung an der Innenseite der Außenwandung 14b festgelegt. Es ist unschwer zu erkennen, dass sich durch diese Bauweise ein noch kompakterer Aufbau ergibt bzw. die Möglichkeit ergibt, Medienspeicher größeren Durchmessers bei unveränderten Außenmaßen in der Austragvorrichtung zu verwenden.

Bei der Ausgestaltung gemäß der Fig. 5b findet ebenfalls ein Bauteilstrang 20' Anwendung, der im Wesentlichen mit dem Bauteilstrang 20 der Fig. 3 übereinstimmt. Dieser ist jedoch mehrfach gefaltet, so dass sich ein Schichtverbund ergibt, der vollständig im Bereich des Sichtfensters 14c angeordnet ist. Das Fenster für den Sensor 50 an der Gehäuseinnenwandung 14a ist daher ebenfalls zum Sichtfenster 14c hin ausgerichtet angeordnet.

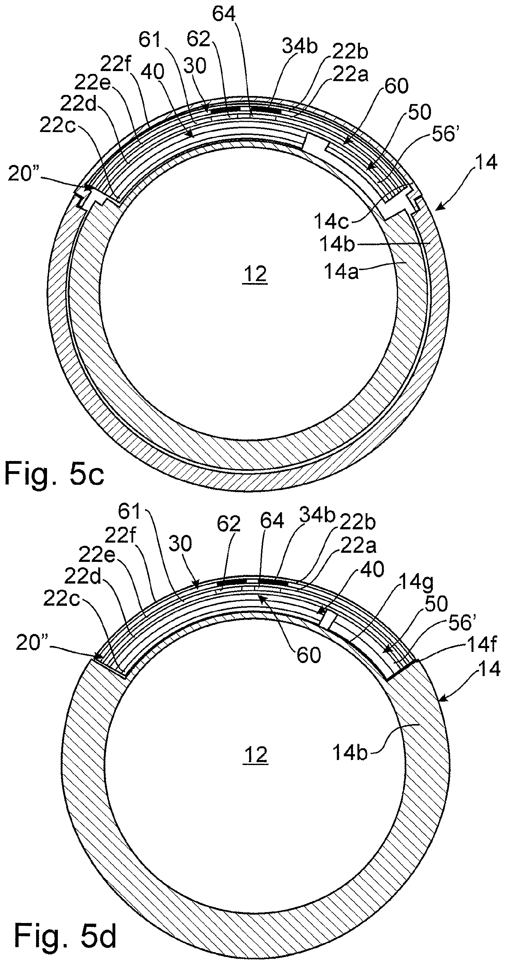

Fig. 5c zeigt eine Variation hierzu. Bei dieser sind die Unterbaugruppen 30, 40, 50, 60 unmittelbar in Form eines Schichtverbundes 20" hergestellt, so dass sich keine Knickkanten ergeben. Stattdessen sind die jeweiligen aktiven Bauteile der Unterbaugruppen, die durch Folienlagen 22c, 22d, 22e, 22f gegeneinander bzw. gegenüber einer Umgebung isoliert sind, miteinander durch nicht dargestellte Durchbrechungen in den Folienlagen miteinander verbunden. Ein weiterer Unterschied zur Ausführungsform der Fig. 5b liegt darin, dass die Sensorunterbaugruppe 50 vorliegend einen drucktechnisch aufgebrachten Kapazitätssensor 56' aufweist. Dieser ist geeignet, eine Kapazitätsänderung zu erfahren, wenn der Medienspeicher 8 im Aufnahmeraum 12 bewegt wird. Da kein direkter Berührkontakt zwischen dem Medienspeicher 8 und der Sensor-unterbaugruppe 50 erforderlich ist, ist die Innenwandung 14a bei dieser Ausgestaltung umfänglich geschlossen und weist abweichend von der Ausgestaltung der Fig. 3 und 5b kein hierfür vorgesehenes Fenster auf.

Bei der Ausgestaltung gemäß der Fig. 5d ist der Grundaufbau der Baugruppe 20" mit dem der Fig. 5c vergleichbar. Die Besonderheit liegt daran, dass lediglich eine Außenwandung 14b vorgesehen ist, die über einen Winkelbereich von etwa 135° eine Vertiefung 14f aufweist, in welche diese Baugruppe 20" eingefügt ist. Die Verbindung der Außenwandung 14c mit der Baugruppe 20" erfolgt dabei vorzugsweise über eine Klebeschicht 14g.

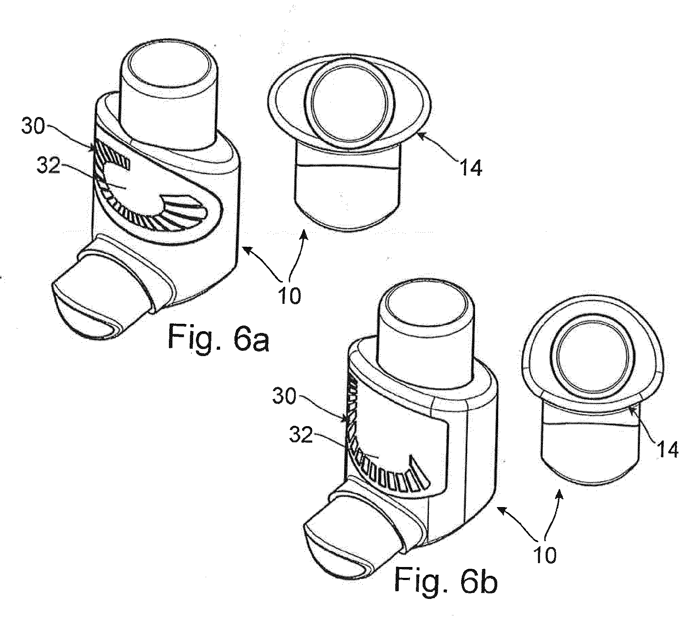

Die Fig. 6a und 6b zeigen alternative Außenformen einer Austragvorrichtung 10. Im Falle der Darstellung der Fig. 6a ist ein ovaler Querschnitt des Hauptabschnitts 14 vorgesehen. Im Falle der Ausgestaltung gemäß Fig. 6b ist ein einseitig abgeflachter Querschnitt vorgesehen. Hierdurch wird erreicht, dass der Biegeradius im Bereich der Anzeigevorrichtung 20 größer sein kann, was fertigungstechnische oder montagetechnische Vorteile und insbesondere Kosteneinsparungen mit sich bringen kann.

## Patentansprüche

1. Austragvorrichtung (10) für pharmazeutische Medien mit
- einem Gehäuse (14, 16), welches einen Aufnahmeraum (12) zur Aufnahme eines Medienspeichers (8) zumindest zum Teil umgibt,
- einer am Gehäuse (14, 16) vorgesehenen Austragöffnung (17) zur Verabreichung des Mediums aus dem Mediumsspeicher (8),
- einer elektronischen Schaltung (60), die zur Erfassung von Austragvorgängen ausgebildet ist, und
- eine Anzeigevorrichtung (30), die einen Anzeigebereich (32) zur Darstellung von Statusinformationen aufweist,
wobei
- die elektronische Schaltung (60) mit der Anzeigevorrichtung (30) verbunden ist,
- das Gehäuse (14, 16) zumindest abschnittsweise eine konvex gewölbte Außenform aufweist,
- der Anzeigebereich (32) der Anzeigevorrichtung (30) entsprechend der konvex gewölbten Außenform des Gehäuses (14, 16) eine gebogene Formgebung aufweist, **gekennzeichnet dadurch, dass** die Anzeigevorrichtung (30) ein Kunststoffsubstrat (22a) aufweist, auf welchem Anzeigesegmente (34a, 34b) vorgesehen sind, die durch Drucktechnik oder Beschichtungstechnik aufgebracht wurden, und
- die mindestens eine gewölbte Leiterplatte (61) als flexible Leiterplatte (61) ausgebildet ist, und das Kunststoffsubstrat (22a) als Träger aufweist, wobei Leiterbahnen (24, 25, 26, 27) auf diesem Kunststoffsubstrat (22a) in Form von Metallfolien oder in Form von aufgedruckten Leiterbahnen (24, 25, 26, 27) vorgesehen sind.

2. Austragvorrichtung
**dadurch gekennzeichnet, dass**
die gewölbte Leiterplatte (61) als FPCB-Leiterplatte ausgebildet ist,

3. Austragvorrichtung nach Anspruch 1 oder 2,
**dadurch gekennzeichnet, dass**
ein Mikroprozessor (62) zur Erfassung von Austragvorgängen und/oder zur Ansteuerung der Anzeigevorrichtung (30) mittels Drucktechnik auf dem Kunststoffsubstrat aufgebracht ist.

4. Austragvorrichtung nach einem der vorstehenden Ansprüche,
**gekennzeichnet durch**
eine Batterie (40) zur Versorgung der elektronischen Schaltung (60) und/oder der Anzeigevorrichtung (30) mit elektrischer Energie, wobei diese Batterie als Flachbatterie ausgebildet und hinter einer gewölbten Außenwandung (14b) des Gehäuses (14, 16) angeordnet ist, wobei sie entsprechend der gewölbten Außenwandung eine gebogene Formgebung aufweist.

5. Austragvorrichtung nach einem der vorstehenden Ansprüche,
**gekennzeichnet durch**
einen Sensor (50) zur Erfassung eines Austragvorgangs, wobei der Sensor (50) vorzugsweise als Kraftsensor oder als kapazitiver Sensor ausgebildet ist.

6. Austragvorrichtung nach Anspruch 5,
**dadurch gekennzeichnet, dass**
der Sensor vorzugsweise auf dem Kunststoffsubstrat (22a) aufgedruckt ist.

7. Austragvorrichtung nach einem der vorstehenden Ansprüche,
**dadurch gekennzeichnet, dass**
das Gehäuse (14, 16) eine nach außen gewandte Außenschale (14b) und eine zum Aufnahmeraum (12) gewandte Innenschale (14a) aufweist, die gemeinsamen einen dazwischenliegenden Elektronikraum (14d) begrenzen, wobei in diesem Elektronikraum (14d) die Anzeigevorrichtung (30) und/oder die mindestens eine Leiterplatte (61; 61a, 61 b) angeordnet ist.

8. Austragvorrichtung nach einem der Ansprüche 1 bis 7,
**dadurch gekennzeichnet, dass**
das Gehäuse eine nach außen gewandte Außenschale (14b) aufweist, an deren Innenseite die mindestens eine Leiterplatte (61) oder die Batterie (40) vorgesehen ist, wobei zwischen der Leiterplatte (61) oder der Batterie (40) einerseits und dem Aufnahmeraum (12) zur Aufnahme des Medienspeichers (8) keine weiteren Wandungen des Gehäuses (14, 16) vorgesehen sind.

9. Austragvorrichtung nach einem der Ansprüche 1 bis 8,
**dadurch gekennzeichnet, dass**
eine formflexible Baueinheit (20') umfassend die Anzeigevorrichtung (30), die elektronische Schaltung (60), den Sensor (50) und die Batterie (40) außenseitig auf einer Außenwandung (14b) des Gehäuses aufgebracht ist, wobei die Baueinheit (20') vorzugsweise an der Außenwandung festgeklebt ist.

10. Austragvorrichtung nach einem der vorstehenden Ansprüche,
**dadurch gekennzeichnet, dass**
die Anzeigevorrichtung (30) und/oder die Außenwandung (14b, 14c), hinter der die mindestens eine Leiterplatte (61) angeordnet ist, im Bereich eines zylindrischen Gehäuseabschnitts (14) vorgesehen ist, wobei die Querschnittsform dieses zylindrischen Gehäuseabschnitts (14) vorzugsweise eine Kreisform bildet.

11. Austragvorrichtung nach einem der vorstehenden Ansprüche,
**dadurch gekennzeichnet, dass**
mindestens zwei der vier Bauteile aus der Gruppe umfassend die Anzeigevorrichtung (30), die elektronische Schaltung (60), einen Sensor (50) zur Erfassung eines Austragvorgangs und eine Batterie (40) auf einem gemeinsamen und einstückigen Kunststoffsubstrat (22a) aufgebracht sind.

12. Austragvorrichtung nach einem der vorstehenden Ansprüche,
**dadurch gekennzeichnet, dass**
mindestens drei der vier Bauteile aus der Gruppe umfassend die Anzeigevorrichtung (30), die elektronische Schaltung (60), einen Sensor (50) zur Erfassung eines Austragvorgangs und eine Batterie (40) entlang eines verformbaren Bauteilstrangs (20) hintereinander angeordnet sind, wobei dieser Bauteilstrang (20) der Formgebung des zylindrischen Gehäuseabschnitts folgend im Gehäuse angeordnet ist.

13. Austragvorrichtung nach einem der Ansprüche 1 bis 11,
**dadurch gekennzeichnet, dass**
mindestens zwei der vier Bauteile aus der Gruppe umfassend die Anzeigevorrichtung (30), die elektronische Schaltung (60), einen Sensor (50) zur Erfassung eines Austragvorgangs und eine Batterie (40) jeweils eine gebogene Formgebung aufweisen und nebeneinander liegende Schichten eines Schichtverbundes (20'; 20") darstellen.

14. Austragvorrichtung nach einem der vorstehenden Ansprüche,
**dadurch gekennzeichnet, dass**
die Austragvorrichtung (10) als Inhalator (10) mit einem in etwa zylindrischen Hauptabschnitt (14) ausgebildet ist, wobei an einem Ende des Hauptabschnitts (14) ein sich von Hauptabschnitt (14) radial nach außen erstreckendes Mundstück (16) mit Austragöffnung (17) vorgesehen ist und wobei der Hauptabschnitt (14) den zylindrischen Gehäuseabschnitt (14) zur Anordnung der Anzeigevorrichtung (30) und/oder der mindestens einen Leiterplatte (61) bildet.

## Claims

1. Discharge device (10) for pharmaceutical media comprising
- a housing (14, 16), which at least partly surrounds a receptacle space (12) for receiving a medium store (8),
- a discharge opening (17) provided on the housing (14, 16) and serving for administering the medium from the medium store (8),
- an electronic circuit (60) designed for detecting discharge processes, and
- a display device (30) having a display region (32) for representing status information,
wherein
- the electronic circuit (60) is connected to the display device (30),
- the housing (14, 16) has a convexly curved outer form at least in sections,
- the display region (32) of the display device (30) has a curved shaping corresponding to the convexly curved outer form of the housing (14, 16),
- the electronic circuit (60) has a curved printed circuit board (61), which is arranged behind a curved outer wall (14b) of the housing (14, 16) and has a curved shaping corresponding to the curved outer wall (14b), **characterized in that**:
the display device (30) has a plastic substrate (22a), on which display segments (34a, 34b) are provided, which were applied by printing technology or coating technology, and
- the at least one curved printed circuit board (61) is embodied as a flexible printed circuit board (61), and has the plastic substrate (22a) as a carrier, wherein conductor tracks (24, 25, 26, 27) are provided on said plastic substrate (22a) in the form of metal films or in the form of printed conductor tracks (24, 25, 26, 27).

2. Discharge device
**characterized in that**
the curved printed circuit board (61) is embodied as an FPCB printed circuit board.

3. Discharge device according to Claim 1 or 2,
**characterized in that**
a microprocessor (62) for detecting discharge processes and/or for driving the display device (30) is applied on the plastic substrate by means of printing technology.

4. Discharge device according to any of the preceding claims,
**characterized by**
a battery (40) for supplying the electronic circuit (60) and/or the display device (30) with electrical energy, wherein said battery is embodied as a flat battery and is arranged behind a curved outer wall (14b) of the housing (14, 16), wherein it has a curved shaping corresponding to the curved outer wall.

5. Discharge device according to any of the preceding claims,
**characterized by**
a sensor (50) for detecting a discharge process, wherein the sensor (50) is preferably embodied as a force sensor or as a capacitive sensor.

6. Discharge device according to Claim 5,
**characterized in that**
the sensor is preferably printed on the plastic substrate (22a).

7. Discharge device according to any of the preceding claims,
**characterized in that**
the housing (14, 16) has an outwardly facing outer shell (14b) and an inner shell (14a) facing the receptacle space (12), which together delimit an intervening electronics space (14d), wherein the display device (30) and/or the at least one printed circuit board (61; 61a, 61b) are/is arranged in said electronics space (14d).

8. Discharge device according to any of Claims 1 to 7,
**characterized in that**
the housing has an outwardly facing outer shell (14b), on the inner side of which the at least one printed circuit board (61) or the battery (40) is provided, wherein no further walls of the housing (14, 16) are provided between the printed circuit board (61) or the battery (40), on the one hand, and the receptacle space (12) for receiving the medium store (8).

9. Discharge device according to any of Claims 1 to 8,
**characterized in that**
a dimensionally flexible structural unit (20') comprising the display device (30), the electronic circuit (60), the sensor (50) and the battery (40) is applied on the outer side on an outer wall (14b) of the housing, wherein the structural unit (20') is preferably fixedly adhesively bonded to the outer wall.

10. Discharge device according to any of the preceding claims,
**characterized in that**
the display device (30) and/or the outer wall (14b, 14c), behind which the at least one printed circuit board (61) is arranged, are/is provided in the region of a cylindrical housing section (14), wherein the cross-sectional form of said cylindrical housing section (14) preferably forms a circular form.

11. Discharge device according to any of the preceding claims,
**characterized in that**
at least two of the four components from the group comprising the display device (30), the electronic circuit (60), a sensor (50) for detecting a discharge process and a battery (40) are applied on a common and integral plastic substrate (22a).

12. Discharge device according to any of the preceding claims,
**characterized in that**
at least three of the four components from the group comprising the display device (30), the electronic circuit (60), a sensor (50) for detecting a discharge process and a battery (40) are arranged one behind another along a deformable component strand (20), wherein said component strand (20) is arranged in the housing in a manner following the shaping of the cylindrical housing section.

13. Discharge device according to any of Claims 1 to 11,
**characterized in that**
at least two of the four components from the group comprising the display device (30), the electronic circuit (60), a sensor (50) for detecting a discharge process and a battery (40) in each case have a curved shaping and constitute adjacent layers of a layer composite assembly (20'; 20").

14. Discharge device according to any of the preceding claims,
**characterized in that**
the discharge device (10) is embodied as an inhaler (10) having an approximately cylindrical main section (14), wherein a mouthpiece (16) extending radially outward from main section (14) and having a discharge opening (17) is provided at one end of the main section (14), and wherein the main section (14) forms the cylindrical housing section (14) for the arrangement of the display device (30) and/or the at least one printed circuit board (61).

## Revendications

1. Dispositif de distribution (10) pour des médias pharmaceutiques, comprenant :
- un boîtier (14, 16), lequel entoure, tout au moins en partie, un compartiment de réception (12) destiné au logement d'un réservoir à médias (8) ;
- un orifice de distribution (17) prévu au niveau du boîtier (14, 16) en vue de l'administration du média à partir du réservoir à médias (8) ;
- un circuit électronique (60) conçu en vue de la détection des processus de distribution ; et
- un dispositif d'affichage (30) qui présente une zone d'affichage (32), laquelle est destinée à la représentation des informations d'état,
dans lequel
- le circuit électronique (60) est raccordé au dispositif d'affichage (30) ;
- le boîtier (14, 16) présente, tout au moins par sections, une forme extérieure dont la courbure est convexe ;
- la zone d'affichage (32) du dispositif d'affichage (30) présente un façonnage coudé qui correspond à la forme extérieure de courbure convexe du boîtier (14, 16) ;
le circuit électronique (60) présente une carte de circuits imprimés (61) de forme incurvée qui est disposée à l'arrière d'une paroi extérieure (14b) incurvée du boîtier (14, 16) et qui présente un façonnage coudé correspondant à la paroi extérieure (14b) de forme incurvée,
**caractérisé en ce que**
le dispositif d'affichage (30) présente un substrat en plastique (22a), sur lequel sont prévus des segments d'affichage (34a, 34b) qui ont été appliqués par l'intermédiaire d'une technique d'impression ou d'une technique de revêtement ;
- l'au moins une carte de circuits imprimés (61), laquelle est de forme incurvée, est conçue sous la forme d'une carte de circuits imprimés (61) flexible et présente le substrat en plastique (22a) sous la forme d'un support, dans lequel des pistes conductrices (24, 25, 26, 27) sont prévues sur ce substrat en plastique (22a), lesquelles se présentent sous la forme de feuilles en métal ou sous la forme de pistes conductrices (24, 25, 26, 27) imprimées.

2. Dispositif de distribution
**caractérisé en ce que**
la carte de circuits imprimés (61) de forme incurvée est conçue sous la forme d'une carte FPCB (carte de circuits imprimés flexible).

3. Dispositif de distribution selon la revendication 1 ou 2,
**caractérisé en ce que**
un microprocesseur (62) est mis en place sur le substrat en plastique au moyen d'une technique d'impression, en vue de la détection des processus de distribution et/ou en vue de la commande du dispositif d'affichage (30).

4. Dispositif de distribution selon l'une des revendications précédentes,
**caractérisé par**
une batterie (40) destinée à l'alimentation du circuit électronique (60) et/ou du dispositif d'affichage (30) avec une énergie électrique, dans lequel cette batterie est conçue sous la forme d'une batterie plate et est disposée à l'arrière d'une paroi extérieure (14b) incurvée du boîtier (14, 16), dans lequel elle présente un façonnage coudé qui correspond à la paroi extérieure de forme incurvée.

5. Dispositif de distribution selon l'une des revendications précédentes,
**caractérisé par**
un capteur (50) destiné à la détection d'un processus de distribution, dans lequel le capteur (50) est conçu de préférence sous la forme d'un capteur de force ou sous la forme d'un capteur capacitif.

6. Dispositif de distribution selon la revendication 5,
**caractérisé en ce que**
le capteur est imprimé, de préférence, sur le substrat en plastique (22a) .

7. Dispositif de distribution selon l'une des revendications précédentes,
**caractérisé en ce que**
le boîtier (14, 16) présente une coque extérieure (14b) qui est orientée vers l'extérieur et une coque intérieure (14a) qui est orientée vers le compartiment de réception (12), lesquelles coques délimitent ensemble un compartiment des composants électroniques (14d) qui se situe entre les deux coques, dans lequel le dispositif d'affichage (30) et/ou l'au moins une carte de circuits imprimés (61 ; 61a, 61b) sont disposés dans ce compartiment des composants électroniques (14d).

8. Dispositif de distribution selon l'une des revendications 1 à 7,
**caractérisé en ce que**
le boîtier présente une coque extérieure (14b) orientée vers l'extérieur, au niveau de la face intérieure de laquelle est prévue l'au moins une carte de circuits imprimés (61) ou la batterie (40), dans lequel aucune autre paroi du boîtier (14, 16) n'est prévue entre la carte de circuits imprimés (61) ou la batterie (40), d'une part, et le compartiment de réception (12) destiné au logement du réservoir à médias (8), d'autre part.

9. Dispositif de distribution selon l'une des revendications 1 à 8,
**caractérisé en ce que**
une unité structurelle (20') dont la forme est flexible et comprenant le dispositif d'affichage (30), le circuit électronique (60), le capteur (50) et la batterie (40) est mise en place, du côté extérieur, sur une paroi extérieure (14b) du boîtier, dans lequel l'unité structurelle (20') est, de préférence, collée fixement à la paroi extérieure.

10. Dispositif de distribution selon l'une des revendications précédentes,
**caractérisé en ce que**
le dispositif d'affichage (30) et/ou la paroi extérieure (14b, 14c) sont disposés à l'arrière de l'au moins une carte de circuits imprimés (61) et sont prévus au niveau d'une section de boîtier (14) cylindrique, dans lequel la forme de section transversale de cette section de boîtier (14) cylindrique forme de préférence une forme circulaire.

11. Dispositif de distribution selon l'une des revendications précédentes,
**caractérisé en ce que**
au moins deux des quatre éléments structurels constitués par le groupe comprenant le dispositif d'affichage (30), le circuit électronique (60), un capteur (50) destiné à la détection d'un processus de distribution et une batterie (40) sont mis en place sur un substrat en plastique (22a) commun, lequel est moulé d'une seule pièce.

12. Dispositif de distribution selon l'une des revendications précédentes,
**caractérisé en ce que**
au moins trois des quatre éléments structurels constitués par le groupe comprenant le dispositif d'affichage (30), le circuit électronique (60), un capteur (50) destiné à la détection d'un processus de distribution et une batterie (40) sont disposés les uns à la suite des autres, le long d'un tronçon déformable de l'élément structurel (20), dans lequel ce tronçon de l'élément structurel (20) est disposé dans le boîtier en suivant le façonnage de la section de boîtier cylindrique.

13. Dispositif de distribution selon l'une des revendications 1 à 11,
**caractérisé en ce que**
au moins deux des quatre éléments structurels constitués par le groupe comprenant le dispositif d'affichage (30), le circuit électronique (60), un capteur (50) destiné à la détection d'un processus de distribution et une batterie (40) présentent respectivement un façonnage coudé et représentent des couches d'un composite en couches (20' ; 20"), lesquelles couches sont mises en place les unes à côté des autres.

14. Dispositif de distribution selon l'une des revendications précédentes,
**caractérisé en ce que**
le dispositif de distribution (10) est conçu sous la forme d'un inhalateur (10) comprenant une section principale (14) quelque peu cylindrique, dans lequel un embout (16), qui est doté d'un orifice de distribution (17) et qui s'étend de manière radiale vers l'extérieur depuis la section principale (14), est prévu au niveau d'une extrémité de la section principale (14) et dans lequel la section principale (14) forme la section de boîtier (14) cylindrique en vue de l'agencement du dispositif d'affichage (30) et/ou de l'au moins une carte de circuits imprimés (61).
